# EUROPEAN PATENT APPLICATION

(11) **EP 1 690 856 A1**
(43) Date of publication of application: **16.08.2006**
(21) Application number: 04819432.8
(22) Date of filing: 26.11.2004
(51) Int. Cl.: C07D 239/94, C07D 239/88

(54) **QUINAZOLINE DERIVATIVE AND PROCESS FOR PRODUCING THE SAME**

(30) Priority: 28.11.2003 JP 2003398442
(71) Applicant: Mitsubishi Pharma Corporation, Osaka-shi, Osaka 541-0046 (JP)
(72) Inventor: IWAMURA, Hiroshi, Mitsubishi Pharma Corporation, Tokyo 103-8405 (JP); NAKA, Takashi, Mitsubishi Pharma Corporation, Tokyo 103-8405 (JP)
(74) Representative: Gerstberger, Gisela
(86) International application number: PCT/JP2004/017564
(87) International publication number: WO 2005/051924

(57) **Abstract**

Object: To offer novel quinazoline derivatives, which are useful as intermediates in the production of agents for the treatment and prevention of cancer, and an industrially useful method for manufacture of the same. Means: The quinazoline derivative represented by general formula (I) below: 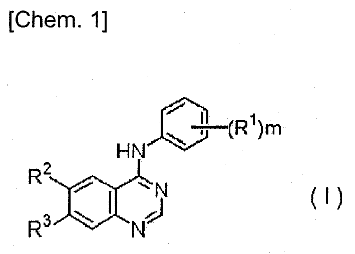 (in the formula, each of the symbols is the same as described in the specification)
or a salt thereof, or a hydrate or solvate thereof, and a method for using the same to produce novel quinazoline derivatives.

## Description

### TECHNICAL FIELD

The present invention relates to novel quinazoline derivatives that are useful as synthesis intermediates for pharmaceuticals and physiologically active substances, particularly as intermediates in the production of agents for the treatment and prevention of cancer, and to a method for production of the same.

### BACKGROUND ART

Quinazoline derivatives represented by general formula (1a): 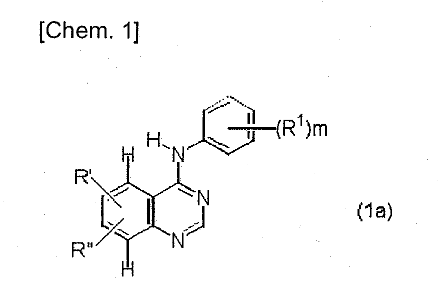 {The reference symbols in the formula are such that, for example, m denotes an integer of 1 or 2, R¹ denotes a halogen atom, either R' or R" denotes: 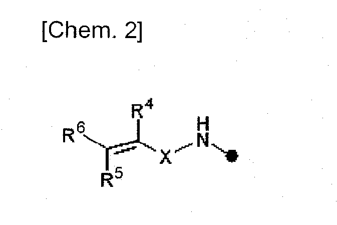 (In the formula, X denotes -C(O)-, and R⁴, R⁵ and R⁶ each individually denotes a C₁ to C₅ alkyl that may be substituted.), and the remaining one of R² and R³ denotes: 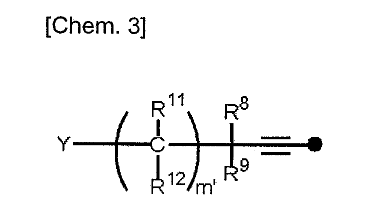 [In the formula, m' denotes an integer from 0 to 3, R⁸ and R⁹ each individually denotes a C₁ to C₅ alkyl that may be substituted, R¹¹ and R¹² each individually denotes a hydrogen atom or C₁ to C₅ alkyl, Y denotes: 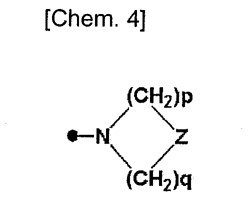 (In the formula, p and q each individually denotes an integer of 2 or 3, and Z denotes -O- or a carbonyl.)]}
exhibit inhibitory action with respect to both the EGF receptor and the HER2 receptor (dual inhibitory actions), and are known to have cancer cell growth inhibitory actions (see Patent Document 1).

A known general synthesis example for this type of quinazoline derivative involves, for example, a reaction between a quinazoline compound, wherein R' is a nitro group or amino group and R" is a chlorine atom or bromine atom, and a substituted alkyne compound in the presence of a metal reagent such as Pd to produce an alkynyl quinazoline derivative, followed by conversion to the corresponding amino compound, followed by derivation of a vinylamide.

In the above method, the reaction in which the quinazoline compound and alkyne compound bond together is carried out under high-temperature conditions.

Furthermore, in the reaction that generates a carbon-carbon bond using a Pd catalyst, various investigations have been carried out regarding reaction conditions in the vicinity of room temperature, but all of these use costly unstable reagents (see Non-Patent Document 1). For this reason, there is need for the discovery of a simple technique for obtaining and handling the reagent in order to carry out this reaction on an industrial scale.

In addition to the above, it has not been possible to achieve adequate yields in the step in which the aminoquinazoline derivative is converted to the vinylamide quinazoline derivative.

The yield of vinylamidation of the amino groups of the aminoquinazoline derivative is generally known to be low (see Non-Patent Documents 2 and 3). It has been hypothesized that polymerization by way of the vinyl groups may be a factor, and details regarding a similar reaction with an oxazolidinone derivative have been reported (see Non-Patent Documents 4 and 5).

In light of the above, there is need for the discovery of a synthesis method for alkynyl quinazoline derivatives as well as a technique for increasing the yield at which vinylamide groups are created in order to produce vinylamidoquinazoline derivatives on an industrial scale.

Patent Document 1: International Publication WO 02/066445.

Non-Patent Document 1: *Org. Lett.,* 2000, 2, 1729.

Non-Patent Document 2: *J. Med. Chem.,* 1999, 42, 1803.

Non-Patent Document 3: *J. Med. Chem.,* 2000, 43, 1387.

Non-Patent Document 4: *Tetrahedron Lett.,* 1996, 52, 13523.

Non-Patent Document 5: *J. Org. Chem.,* 1995, 60, 2271.

### DISCLOSURE OF THE INVENTION

### Problems to Be Solved by the Invention

It is necessary to solve problems such as those described above in order to industrially produce quinazoline derivatives of the type represented by general formula (1a).

Specifically, an object of the present invention is to provide an industrially advantageous production method and a novel synthesis intermediate in this production method by means of (1) relaxing the conditions for the reaction between the quinazoline compound and alkyne compound, and (2) creating vinylamide groups at higher yields.

### Means for Solving the Problems

In the Pd catalyst oxidative addition reaction, it is known that the corresponding iodo compounds have higher reactivity than the bromo compounds and chloro compounds for organic halides substituted with SP2 carbons (*Palladium Reagent and Catalysts,* Tsuji, J. Eds.; John Wiley & Sons: Chichester, 1995; Chapter 1).

The present inventors investigated reactions between quinazoline derivatives represented by general formula (I) below: 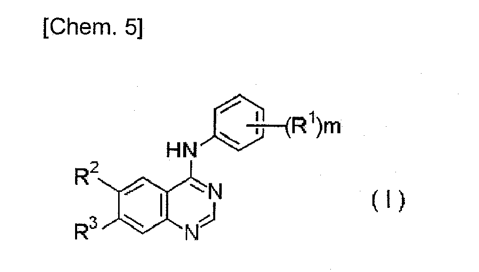 (In the formula, m denotes an integer from 0 to 3, R¹ denotes a hydrogen atom, halogen atom, hydroxy group, cyano group, nitro group, trifluoromethyl group, C₁ to C₅ alkyl group, C₁ to C₅ alkoxy group, -S(O)_{f}R¹² (In the formula, f denotes an integer from 0 to 2 and R¹² denotes a C₁ to C₅ alkyl group.), -NR¹³R¹⁴ (In the formula, R¹³ and R¹⁴ each individually denotes a hydrogen atom, C₁ to C₅ alkyl group, C₁ to C₅ alkanoyl group, or C₁ to C₅ alkylsulfonyl group.), C₂ to C₅ alkenyl group, or C₂ to C₅ alkynyl group, and either one of R² and R³ denotes general formula (II) below: 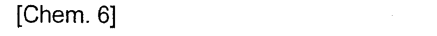 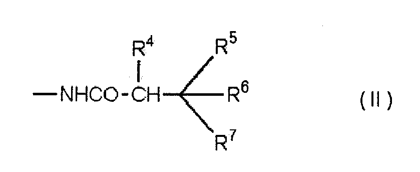 (In the formula, R⁴, R⁵ and R⁶ each individually denotes a hydrogen atom, C₂ to C₅ alkyl group that may have substituents, C₇ to C₁₂ aralkyl group that may have substituents, or C₆ to C₁₀ aryl group that may have substituents, R⁷ denotes -SO₂R¹⁵, -SOR¹⁵, or -OR¹⁵ (In the formula, R¹⁵ denotes a C₁ to C₅ alkyl group that may have substituents, C₇ to C₁₂ aralkyl group that may have substituents, or C₆ to C₁₀ aryl group that may have substituents) and the remaining one of R² and R³ denotes an iodine atom. Note that each R¹ may be the same or different when m denotes 2 or 3.)
and alkyne compounds represented by general formula (VII): 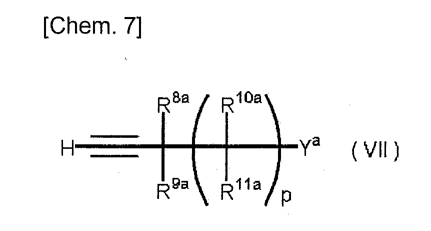 (In the formula, R^{8a} and R^{9a} each individually denotes a hydrogen atom, or a C₁ to C₅ alkyl group that may be substituted with a hydroxyl group or C₁ to C₅ alkoxy group, p denotes an integer from 0 to 3, R^{10a} and R^{11a} each individually denotes a hydrogen atom or C₁ to C₅ alkyl group, Y^{a} denotes a hydrogen atom, hydroxy group, C₁ to C₅ alkoxy group, C₁ to C₅ alkanoyloxy group, piperazin-1-yl that has a C₁ to C₅ alkyl group that may be substituted at the 4-position, or an amino that is disubstituted with C₁ to C₅ alkyls that may be substituted).

As a result, by using the corresponding iodo compound, a coupling reaction was successfully carried out between the quinazoline compound (I) and alkyne compound (VII) under conditions that were more industrially advantageous than those with the chloro compound or bromo compound.

Furthermore, the present inventors attempted to improve yields by suppressing polymerization of the vinylamide quinazoline derivative or the like during production of the vinylamide quinazoline derivative. Specifically, the conversion reaction was successfully carried out at high yield and under mild conditions, and the present invention was realized as a result of investigations involving the production of a quinazoline derivative substituted at the β-position with a labile functional group such as compound represented by general formula (VIII) 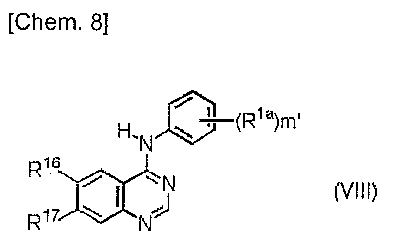 {In the formula, m' and R^{1a} are the same as m and R¹ in general formula (I) above, and either one of R¹⁶ and R¹⁷ denotes general formula (IX) below: 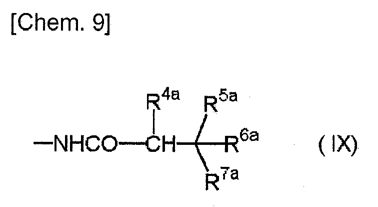 [In the formula, R^{4a}, R^{5a} and R^{6a} are the same as in general formula (II) above, and R^{7a} denotes -SO₂R¹⁵, -SOR¹⁵, or -OR¹⁵ (In the formula, R¹⁵ denotes a C₁ to C₅ alkyl group that may have substituents, a C₇ to C₁₂ aralkyl group that may have substituents, or a C₆ to C₁₀ aryl group that may have substituents.).], and the other of R¹⁶ and R¹⁷ denotes general formula (X) below: 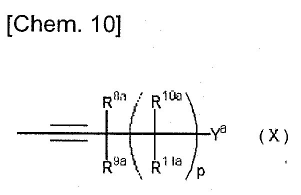 (In the formula, each of the symbols is the same as in general formula (VII) above.).}, followed by conversion to the vinylamidoquinazoline derivative represented by general formula (XI): 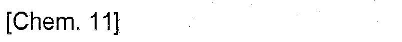 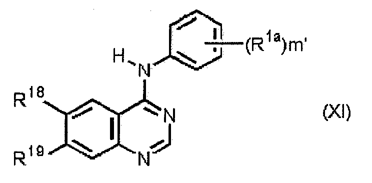 [In the formula, m' and R^{1a} are the same as in general formula (VIII) above, either one of R¹⁸ and R¹⁹ denotes -NHCO-CR^{4a}=CR^{5a}R^{6a} (In the formula, R^{4a}, R^{5a} and R^{6a} are the same as in general formula (IX) above.), and the other of R¹⁸ and R¹⁹ denotes general formula (XII) below: 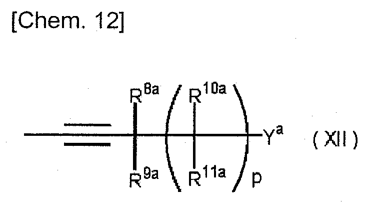 (In the formula, each of the symbols is the same as in general formula (X) above.).].

### Effect of the Invention

By virtue of the present invention, a novel quinazoline derivative can be provided for use as an intermediate in the production of pharmaceutical products such as cancer drugs. Furthermore, the quinazoline derivative can be used in order to provide an efficient production method for pharmaceutical products.

### BEST MODE FOR CARRYING OUT THE INVENTION

Specifically, the gist of the present invention relates to (1) to (24) below.

(1) A quinazoline derivative represented by general formula (I) below, or a salt thereof, or a hydrate or solvate thereof: 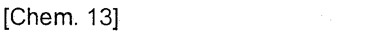 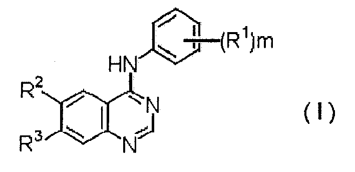 [In the formula, m denotes an integer from 0 to 3, R¹ denotes a hydrogen atom, halogen atom, hydroxy group, cyano group, nitro group, trifluoromethyl group, C₁ to C₅ alkyl group, C₁ to C₅ alkoxy group, -S(O)_{f}R¹² (in the formula, f denotes an integer from 0 to 2, R¹² denotes a C₁ to C₅ alkyl group), -NR ¹³R¹⁴ (in the formula, R¹³ and R¹⁴ each individually denotes a hydrogen atom, C₁ to C₅ alkyl group, C₁ to C₅ alkanoyl group, or C₁ to C₅ alkylsulfonyl group), C₂ to C₅ alkenyl group, or C₂ to C₅ alkynyl group, and either one of R² and R³ denotes general formula (II) below 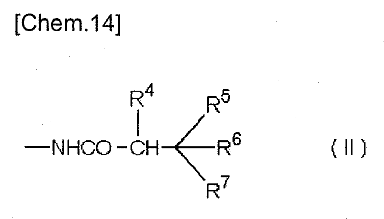 (In the formula, R⁴, R⁵ and R⁶ each individually denotes a hydrogen atom, C₁ to C₅ alkyl group that may have substituents, C₇ to C₁₂ aralkyl group that may have substituents, or C₆ to C₁₀ aryl group that may have substituents, R⁷ denotes -SO₂R¹⁵, -SOR¹⁵, or -OR¹⁵ (in the formula, R¹⁵ denotes a C₁ to C₅ alkyl group that may have substituents, C₇ to C₁₂ aralkyl group that may have substituents, or C₆ to C₁₀ aryl group that may have substituents.) and the remaining one of R² and R³ denotes an iodine atom or general formula (III) below: 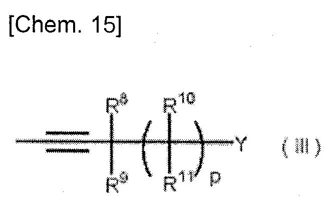 (In the formula, R⁸ and R⁹ each individually denotes a hydrogen atom, or a C₁ to C₅ alkyl group that may be substituted with a hydroxyl group or C₁ to C₅ alkoxy group, p denotes an integer from 0 to 3, R¹⁰ and R¹¹ each individually denotes a hydrogen atom or C₁ to C₅ alkyl group, Y denotes a hydrogen atom, hydroxyl group, C₁ to C₅ alkoxy group, C₁ to C₅ alkanoyloxy group, piperazin-1-yl that has a C₁ to C₅ alkyl group that may be substituted at the 4-position, or an amino that is di-substituted with C₁ to C₅ alkyls that may be substituted.), and herein, when m denotes 2 or 3, R¹ may be the same or different.].

(2) The quinazoline derivative, salt thereof, or hydrate or solvate thereof according to (1) above, wherein m is 2, R¹ is a halogen atom, R² is -NHCO-CH₂-CH₂-R⁷ (in the formula, R⁷ denotes a methylsulfonyl group, benzenesulfonyl group, phenyloxy group, phenylthio group, or methylthio group), and R³ is an iodine atom or general formula (IV) below: 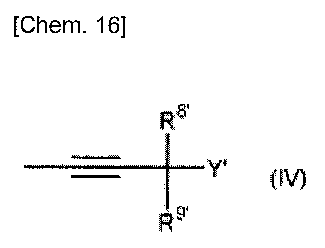 (In the formula, R^{8'} and R^{9'} each individually denotes a hydrogen atom, methyl group, ethyl group, propyl group, or isopropyl group, and Y' denotes a morpholino group or 4-methyl piperazin-1-yl. ).

(3) The quinazoline derivative, salt thereof, or hydrate or solvate thereof according to either (1) or (2) above, selected from a group consisting of the following compounds:
N-{4-[(3-chloro-4-fluorophenyl)amino]-7-iodo-6-quinazolinyl}-3-(phenylsulfonyl)propanamide, N-{4-[(3-chloro-4-fluorophenyl)amino]-7-iodo-6-quinazolinyl}-3-(phenyloxy)propanamide, N-{4-[(3-chloro-4-fluorophenyl)amino]-7-[3-methyl-3-(4-methyl-1-piperazinyl)-1-butynyl]-6-quinazolinyl}-3-(phenylsulfonyl)propanamide, N-{4-[(3-chloro-4-fluorophenyl)amino]-7-[3-methyl-3-(4-methyl-1-piperazinyl)-1-butynyl]-6-quinazolinyl}-3-(phenyloxy)propanamide.

(4) The quinazoline derivative, salt thereof, or hydrate or solvate thereof according to (3) above, wherein the compound is N-{4-[(3-chloro-4-fluorophenyl)amino]-7-iodo-6-quinazolinyl}-3-(phenylsulfonyl)propanamide.

(5) A method for preparing the quinazoline derivative represented by general formula (I) of (1) above [where either of R² and R³ denotes general formula (II) of (1) above, and the other of R² and R³ denotes general formula (III) of (1) above], salt thereof, or hydrate or solvate thereof, by allowing the quinazoline derivative represented by general formula (V) below: 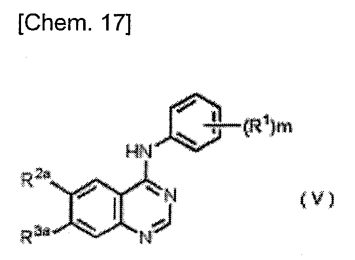 [In the formula, m and R¹ are the same as in (1) above, either one of R^{2a} and R^{3a} is defined the same as in general formula (II) of (1) above, and the other of R^{2a} and R^{3a} denotes an iodine atom.], or salt thereof, or hydrate or solvate thereof to react with a compound represented by general formula (VI) below: 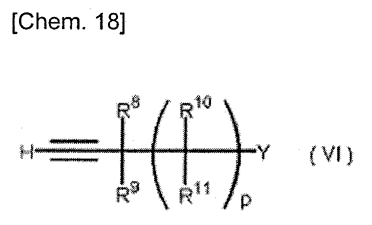 (In the formula, the reference numerals are the same as defined for general formula (III) in (1) above.), or salt thereof, or hydrate or solvate thereof.

(6) The preparation method according to (5) above, wherein m is 2, R¹ is a halogen atom, R^{2a} is -NHCO-CH₂CH₂-R⁷ (in the formula, R⁷ denotes a methylsulfonyl group, benzenesulfonyl group, phenyloxy group, phenylthio group, or methylthio group), and R³ is an iodine atom.

(7) The preparation method according to (5) above, wherein the quinazoline derivative represented by general formula (V) is N-{4-[(3-chloro-4-fluorophenyl)amino]-7-iodo-6-quinazolinyl}-3-(phenylsulfonyl)propanamide or N-{4-[(3-chloro-4-fluorophenyl)amino]-7-iodo-6-quinazolinyl}-3-(phenyloxy)propanamide.

(8) The preparation method according to (7) above, wherein the quinazoline derivative is N-{4-[(3-chloro-4-fluorophenyl)amino]-7-iodo-6-quinazolinyl}-3-(phenylsulfonyl)propanamide.

(9) A method for preparing the compound represented by general formula (III) of (1) above, a pharmaceutically acceptable salt thereof, or a hydrate or solvate thereof, using any of the compounds recited in (1) to (4) above, represented by general formula (VII) 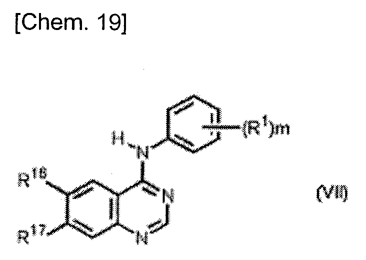 [In the formula, m and R¹ are defined the same as in (1) above, either one of R¹⁶ and R¹⁷ denotes -NHCO-CR⁴=CR⁵R⁶ (in the formula, R⁴, R⁵, and R⁶ are defined the same as in (1) above), and the other one of R¹⁶ and R¹⁷ is.

(10) The preparation method according to (9) above, wherein m is 2, R¹ is a halogen, R² is -NHCO-CH₂CH₂-R⁷, R¹⁶ is -NHCO-CH=CH₂, and R³ and R¹⁷ are general formula (IV) of (2) above.

(11) The preparation method according to (10) above, wherein R^{8'} and R^{9'} each individually is a methyl group, and Y' is 4-methylpiperazin-1-yl.

(12) The preparation method for the compound represented by general formula (VII) of (9) above, salt thereof, or hydrate or solvate thereof comprising the preparation method according to any of (5) to (11) above.

(13) The preparation method according to (12) above, wherein m is 2, R¹ is a halogen, R² is -NHCO-CH₂CH₂-R⁷, R¹⁶ is -NHCO-CH=CH₂, and R³ and R¹⁷ are general formula (IV) of (2) above.

(14) The preparation method according to (12) above, wherein R^{8'} and R^{9'} each individually is a methyl group, and Y' is 4-methylpiperazin-1-yl.

(15) The compound represented by general formula (VIII) below: 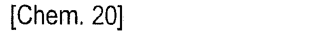 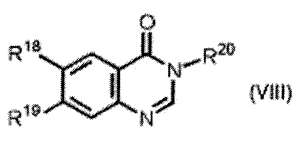 [In the formula, either of R¹⁸ and R¹⁹ denotes a nitro group, amino group, hydroxyamino group, or -NHCO-CH₂CH₂-R^{7'} (in the formula, R^{7'} denotes a methylsulfonyl group, benzenesulfonyl group, phenyloxy group, phenylthio group, or methylthio group), and the remaining one of R¹⁸ and R¹⁹ denotes an iodine atom, and R²⁰ denotes a hydrogen atom, 3,4-dimethoxybenzyl group, 4-methoxybenzyl group, benzyloxymethyl group, or trifluoroacetyl group.],
a salt thereof, or a hydrate or solvate thereof.
(16) A compound, salt thereof, or hydrate or solvate thereof according to (15) above, selected from a group consisting of the following compounds:

7-iodo-3-(4-methoxybenzyl)-6-nitro-4-quinazolinone, 6-amino-7-iodo-3-(4-methoxybenzyl)-4-quinazolinone, N-[7-iodo-3-(4-methoxybenzyl)-4-oxo-3,4-dihydro-6-quinazolinyl]-3-(phenylsulfonyl)propanamide, N-[7-iodo-3-(4-methoxybenzyl)-4-oxo-3,4-dihydro-6-quinazolinyl]-3-(phenyloxy)propanamide, N-(7-iodo-4-oxo-3,4-dihydro-6-quinazolinyl)-3-(phenylsulfonyl)propanamide, and N-(7-iodo-4-oxo-3,4-dihydro-6-quinazolinyl)-3-(phenyloxy)propanamide.

(17) The compound, salt thereof, or hydrate or solvate thereof according to (16) above, wherein the compound is 7-iodo-3-(4-methoxybenzyl)-6-nitro-4-quinazolinone, 6-amino-7-iodo-3-(4-methoxybenzyl)-4-quinazolinone, N-[7-iodo-3-(4-methoxybenzyl)-4-oxo-3,4-dihydro-6-quinazolinyl]-3-(phenylsulfonyl)propanamide, or N-(7-iodo-4-oxo-3,4-dihydro-6-quinazolinyl)-3-(phenylsulfonyl)propanamide.

(18) The preparation method for the compound of general formula (I) in (1) above which uses any of the compounds according to any of (15) to (17) above.

(19) The preparation method according to (18) above, wherein m is 2, R¹ is a halogen atom, R² is -NHCO-CH₂-CH₂-R⁷ (in the formula, R⁷ denotes a methylsulfonyl group, benzenesulfonyl group, phenyloxy group, phenylthio group, or methylthio group), and R³ is an iodine atom or general formula (IV) below: 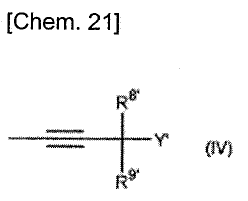 (In the formula, R^{8'} and R^{9'} each individually denotes a hydrogen atom, methyl group, ethyl group, propyl group, or isopropyl group, and Y' denotes a morpholino group or 4-methylpiperazin-1-yl.).

(20) The preparation method according to (18) above, wherein the compound is N-{4-[(3-chloro-4-fluorophenyl)amino]-7-iodo-6-quinazolinyl}-3-(phenylsulfonyl)propanamide, N-{4-[(3-chloro-4-fluorophenyl)amino]-7-iodo-6-quinazolinyl}-3-(phenyloxy)propanamide, N-{4-[(3-chloro-4-fluorophenyl)amino]-7-[3-methyl-3-(4-methyl-1-piperazinyl)-1-butynyl]-6-quinazolinyl}-3-(phenylsulfonyl)propanamide, or N-{4-[(3-chloro-4-fluorophenyl)amino]-7-[3-methyl-3-(4-methyl-1-piperazinyl)-1-butynyl]-6-quinazolinyl}-3-(phenyloxy)propanamide.

(21) The preparation method according to (18) above, wherein the compound is N-{4-[(3-chloro-4-fluorophenyl)amino]-7-iodo-6-quinazolinyl}-3-(phenylsulfonyl)propanamide,

(22) The method for preparing a compound represented by general formula (V) according to (5) above, comprising a step in which a the compound represented by general formula (IX) below: 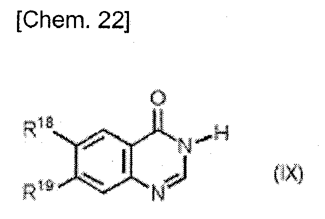 [In the formula, either one of R¹⁸ and R¹⁹ denotes general formula (II) of (1) above, and the remaining one of R¹⁸ and R¹⁹ denotes an iodine atom.]
is chlorinated to produce a compound represented by general formula (X) below: 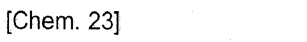 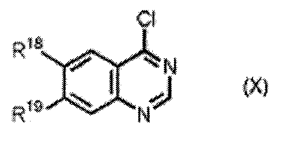 (In the formula, R¹⁸ and R¹⁹ are defined the same as above.), and a step in which a compound represented by general formula (XI) below: 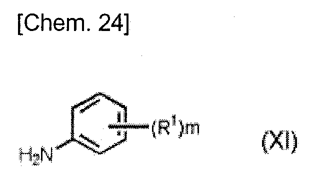 (In the formula, m and R¹ are the defined same as in (1) above.)
is added.

(23) A quinazoline derivative represented by general formula (XII) below: 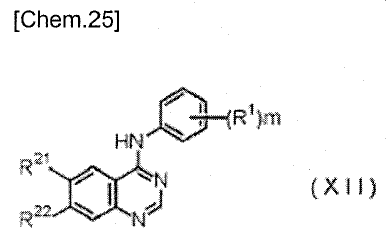 (In the formula, m and R¹ are defined the same as in (1) above, either one of R²¹ and R²² denotes an amino group or nitro group, and the remaining one of R²¹ and R²² denotes an iodine atom.),
a salt thereof, or a hydrate or solvate thereof.

(24) A method for preparing a compound represented by general formula (I) of (1) above, a pharmaceutically acceptable salt thereof, a hydrate or solvate thereof, wherein the nitro group of a compound wherein either one of R²¹ and R²² in general formula (XII) of (23) above is a nitro group and the other one of R²¹ and R²² is an iodine atom is changed to an amino group, whereupon a reaction is allowed to occur with a compound of general formula (XIII) below: 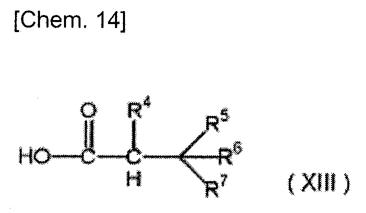 (in the formula, R⁴, R⁵, R⁶ and R⁷ are defined the same as in (1) above).

Hereinbelow, the present invention is defined in detail.

Examples of halogens as defined in the present invention include a fluorine atom, chlorine atom, bromine atom, or iodine atom, examples of C₁ to C₅ alkyl groups include a methyl group, ethyl group, n-propyl group, iso-propyl group, n-butyl group, iso-butyl group, sec-butyl group, tert-butyl group, n-pentyl group, neopentyl group or the like, examples of C₁ to C₅ alkoxy groups include a methoxy group, ethoxy group, n-propoxy group, iso-propoxy group, n-butoxy group, iso-butoxy group, sec-butoxy group, tert-butoxy group, n-pentyloxy group, neopentyloxy group or the like, examples of C₂ to C₅ alkenyl groups include a vinyl group, 1-propenyl group, 2-propenyl group, 1-butenyl group, 2-methylpropen-1-yl group, 2-butenyl group, 1-pentenyl group, 2-pentenyl group or the like, examples of C₂ to C₅ alkynyl groups include an ethynyl group, 1-propynyl group, 1-butynyl group, 1-pentynyl group or the like, examples of C₁ to C₅ alkanoyl groups include a formyl group, acetyl group, propionyl group, butyryl group, isovaleryl group, valeryl group or the like, examples of C₇ to C₁₂ aralkyl groups include a benzyl group, phenylethyl group, phenylpropyl group, naphthylmethyl group or the like, and examples of C₆ to C₁₀ aryl groups include a phenyl group, tolyl group, naphthyl group, mesityl group, xylyl group or the like.

When the phrase "that may have substituents" is used in the definition of substituents in the general formula above, examples of substituents include C₁ to C₆ alkyl groups such as a methyl group, ethyl group, n-propyl group, isopropyl group, n-butyl group, sec-butyl group, isobutyl group, tert-butyl group, n-pentyl group, and n-hexyl group; C₁ to C₆ haloalkyl groups such as a chloromethyl group, bromomethyl group, dichloromethyl group, 1-chloroethyl group, 2-chloroethyl group, 3-chloropropyl group, 4-chlorobutyl group, 5-chloropentyl group, 6-chlorohexyl group, difluoromethyl group, and trifluoromethyl group; C₁ to C₆ alkoxy groups such as a methoxy group, ethoxy group, n-propoxy group, isopropoxy group, n-butoxy group, sec-butoxy group, isobutyloxy group, tert-butyloxy group, n-pentyloxy group, and n-hexyloxy group; and halogen atoms such as a fluorine atom, chlorine atom, and bromine atom.

Salts that may be cited include inorganic acid salts (such as hydrochlorides, sulfates, carbonates, and phosphates), organic acid salts (such as formates, acetates, propionates, lactates, oxalates, fumarates, maleates, citrates, tartrates, benzoates, phthalates, methanesulfonates, p-toluenesulfonates, isethionates, glucuronates, and gluconates), alkali metal salts (such as sodium salts and potassium salts), alkaline earth metal salts (such as magnesium salts and calcium salts), ammonium salts, or salts with organic amines (such as trimethylamine, triethylamine, benzylamine, phenethylamine, monoethanolamine, diethanolamine, tris(hydroxyethylamine), lysine, and arginine).

The quinazoline derivative of the present invention can have various three-dimensional structures. For example, when an asymmetric carbon atom is considered as the center, the absolute configuration may be either (S) or (R), or a racemic form. Pure optical isomer or diastereoisomer configurations, any mixture of these isomers, and racemic forms are all within the scope of the present invention.

Furthermore, the quinazoline derivative represented by general formula (I) above may be present, for example, in the form of a solvate such as a hydrate or in the form of a non-solvate, and the present invention encompasses all of these types of solvate forms that can be used in manufacture.

The compound represented by general formula (I) or (VII) above, for example, can be used as an intermediate in the production of agents for treating and preventing cancer.

An example of a production method according to the present invention is described in further detail below.

Among the quinazoline derivatives represented by general formula (I) above, quinazoline derivatives wherein either one of R² and R³ is represented by general formula (II) above and the other is an iodine atom can be produced, for example, by the method described below (route A).

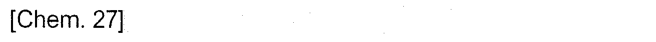

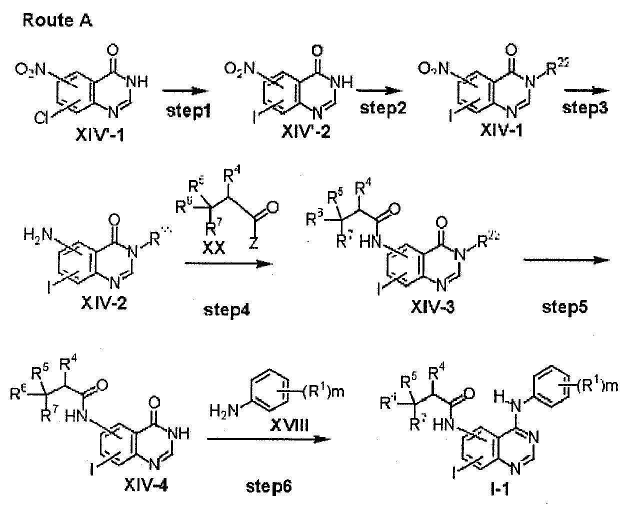

Step 1 involves conversion of the chloro group of quinazoline compound XIV'-1 (In the formula, the nitro group is bonded at either the 6-position or 7-position of the quinazoline ring, and the chloro group is bonded at the other of the 6-position of 7-position of the quinazoline ring. This compound XIV'-1 can be synthesized by the method described in J. *Org. Chem.,* 1975, 40, 356. or the like) into XIV'-2 by means of a halogen exchange reaction.

The halogen exchange reaction can be carried out by using 1 to 100, and preferably 5 to 30, equivalents of quaternary ammonium salt (such as iodobenzyltriethylammonium) or inorganic iodine compound typified by sodium iodide or potassium iodide.

The reaction may be carried out at -50 to 300°C, and preferably 100 to 200°C, in a hydrocarbon-based solvent such as toluene, an ether-based solvent such as tetrahydrofuran (hereinafter "THF"), methyltetrahydrofuran, or ethylene glycol, an ester-based solvent such as ethyl acetate, or isopropyl acetate, or an aprotic polar solvent such as an N,N-dimethylformamide (hereinafter "DMF"), 1 ,3-dimethyl-2-imidazolidinone, 1-methyl-2-pyrrolidone, dimethylsulfoxide (hereinafter "DMSO"), or acetonitrile.

Step 2 is a step in which compound XIV-1 is derived by allowing a reaction to occur at the 3-position of the quinazoline ring of compound XIV'-2.

The reaction can be carried out using 0.8 to 100 equivalents, and preferably 1 to 5 equivalents, of 3,4-dimethoxybenzyl chloride, 3,4-dimethoxybenzyl bromide, 4-methoxybenzyl chloride, 4-methoxybenzyl bromide, benzyl chloromethyl ether, or trifluoroacetic anhydride with respect to compound XIV'-2.

The above reaction can be carried out in the presence or absence of an organic base such as triethylamine, diisopropylethylamine, N-methylmorpholine, pyridine, or 1,4-diazabicyclo[2.2.2]octane (hereinafter "DABCO"), or an inorganic base such as potassium carbonate, sodium hydride, potassium bicarbonate, sodium hydroxide, or potassium phosphate.

The reaction can be carried out at -50 to 300, and preferably 0 to 100°C, in a hydrocarbon-based solvent such as toluene, an ether-based solvent such as THF, methyltetrahydrofuran, or ethylene glycol, an ester-based solvent such as ethyl acetate, or isopropyl acetate, or an aprotic polar solvent such as DMF, 1,3-dimethyl-2-imidazolidinone, 1-methyl-2-pyrrolidone, DMSO, or acetonitrile.

Step 3 is a step in which the nitro group of compound XIV-1 is reduced to derive compound XIV-2. The reaction can be carried out in the presence or absence of 0.1 to 100 equivalents of inorganic acid such as hydrochloric acid or sulfuric acid, or an organic acid such as trifluoroacetic acid or acetic acid, with 0.8 to 100 equivalents, and preferably 1 to 5 equivalents of reduced iron, zinc powder, tin chloride, or the like. Furthermore, reduction can be carried out using 0.001 to 0.05 equivalent of FeCl₃ in the presence of 0.8 to 100 equivalents, and preferably 1 to 5 equivalents, of hydrazine. Under these conditions, the reaction can be carried out in the presence or absence of activated carbon.

The reaction can also be carried out at -50 to 300°C, and preferably 0 to 100°C in a hydrocarbon-based solvent such as toluene, an ether-based solvent such as THF, methyltetrahydrofuran, or ethylene glycol, an ester-based solvent such as ethyl acetate or isopropyl acetate, an aprotic polar solvent such as DMF, 1,3-dimethyl-2-imidazolidinone, 1-methyl-2-pyrrolidone, DMSO, or acetonitrile, or an alcohol-based solvent such as ethanol, methanol, isopropyl alcohol ("IPA" below), propanol, or butanol or without a solvent.

Step 4 involves allowing a reaction to occur between carbonic acid XX (Z = OH) or acid halide thereof and the amino group of compound XIV-2, thereby producing the amide compound XIV-3 (R⁴, R⁵, R⁶ and R⁷ are as described above; reference literature: *Chem. Ber.,* 1924, 57, 202 and J. Am. Chem. Soc., 1952, 74, 1323.).

The reaction is allowed to occur using 0.8 to 100 equivalents, and preferably 1 to 5 equivalents, of XX with respect to compound XIV-2. When the Z in compound XX is -OH, then the reaction is allowed to occur using 0.8 to 100 equivalents, and preferably 1 to 10 equivalents, of a typical peptide bond-forming reagent (such as 1-ethyl-3-(3-dimethylaminopropyl)carbodiimide hydrochloride, N,N'-dicyclohexylcarbodiimide, 1-propylphosphonic acid cyclic anhydride, diethyl chlorophosphate, isobutyl chloroformate, pivaloyl chloride, or thionyl chloride).

Even when the Z of compound XX is -OH or -Cl, the reaction can be carried out in the presence or absence of organic base (such as triethylamine, diisopropylethylamine, pyridine, N-methylmorpholine, or DABCO) or inorganic base (such as potassium carbonate, sodium hydride, potassium bicarbonate, sodium hydroxide, or potassium phosphate).

The reaction can be carried out at -50 to 200°C, and preferably 0 to 50°C in a hydrocarbon-based solvent such as toluene, an ether-based solvent such as THF, methyltetrahydrofuran, or ethylene glycol, an ester-based solvent such as ethyl acetate or isopropyl acetate, or an aprotic polar solvent such as DMF, 1,3-dimethyl-2-imidazolidinone, 1-methyl-2-pyrrolidone, DMSO, or acetonitrile.

Step 5 is a step involving conversion of R²² of compound XIV-3 (a 3,4-dimethoxybenzyl group, 4-methoxybenzyl group, benzyloxymethyl group, or trifluoroacetyl group) into a hydrogen atom. The reaction can be carried out at -50 to 300°C, and preferably 0 to 100°C, in the presence of 0.1 to 100 equivalents of inorganic acid (such as hydrochloric acid or sulfuric acid), or organic acid (such as trifluoroacetic acid or acetic acid), in a hydrocarbon-based solvent such as toluene, an ether-based solvent such as THF, methyltetrahydrofuran, or ethylene glycol, an ester-based solvent such as ethyl acetate or isopropyl acetate, an aprotic polar solvent such as DMF, 1,3-dimethyl-2-imidazolidinone, 1-methyl-2-pyrrolidone, DMSO, or acetonitrile, an alcohol-based solvent such as ethanol, methanol, IPA, propanol, or butanol, or without a solvent.

Step 6 is a reaction in which compound I-1 is produced by allowing 0.8 to 100 equivalents, and preferably 0.9 to 5 equivalents, of aniline derivative XVIII to react after converting the 4-position of the quinazoline ring of compound XIV-4 to a chlorine atom. Chlorination of compound XIV-4 is carried out by using 0.8 to 100 equivalents, and preferably 1 to 10 equivalents, of a chlorination reagent that is commonly used, such as phosphorus oxychloride, thionyl chloride, phosphorus trichloride, phosphorus pentachloride, or N-chlorosuccinic acid imide. Furthermore, the 4-position of the quinazoline ring of compound XIV-4 can be converted to a chlorine atom, even if a quaternary ammonium salt such as tetramethylammonium chloride, tetraethylammonium chloride, benzyltrimethylammonium chloride, or benzyltriethylammonium chloride is used together with the chlorination reagent (Can. *J. Chem.,* 1981, 59, 2601.).

This reaction can also be carried out in the presence or absence of an organic base (such as triethylamine, diisopropylethylamine, pyridine, N-methylmorpholine, or DABCO), or inorganic base (such as potassium carbonate, sodium hydride, potassium bicarbonate, sodium hydroxide, or potassium phosphate).

This reaction can be carried out at -50 to 200°C, and preferably 0 to 100°C, where the reaction solvent is a hydrocarbon-based solvent such as toluene, an ether-based solvent such as THF, methyltetrahydrofuran, or ethylene glycol, an ester-based solvent such as ethyl acetate or isopropyl acetate, or an aprotic polar solvent such as DMF, 1,3-dimethyl-2-imidazolidinone, 1-methyl-2-pyrrolidone, DMSO, or acetonitrile.

Similarly, of the quinazoline derivatives represented by general formula (I) above, quinazoline derivatives in which either of R² and R³ is represented by general formula (II) above and the other is an iodine atom can be produced, for example, in the manner described below (route B).

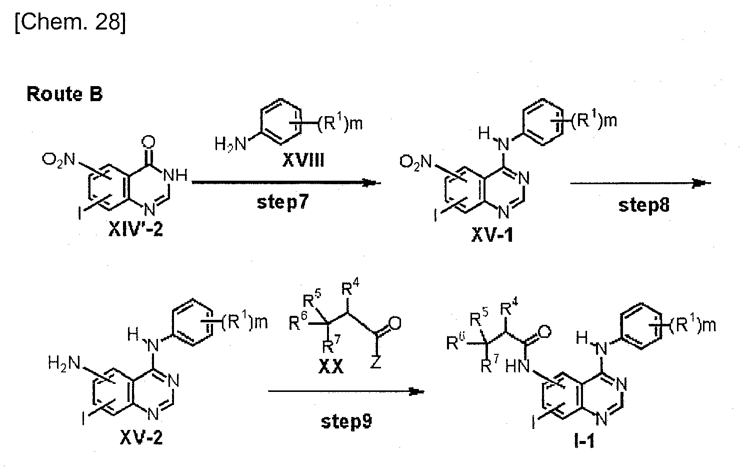

Step 7 is a step in which the aniline derivative XVIII is allowed to react after conversion of the 4-position of the quinazoline ring of compound XIV'-2 to a chlorine atom, thereby producing compound XV-1. In this step, synthesis is possible by the same method as in the process of Step 6.

Step 8 is a step in which the nitro group of compound XV-1 is reduced to derive compound XV-2. In this step, synthesis can be carried out in the same manner as in the process of Step 3.

Step 9 is a step in which carboxylic acid XX (Z=OH) or acid halide thereof is allowed to react with the amino group of compound XV-2, thereby producing an amide I-1. In this step, synthesis is possible by the same method as Step 4.

A quinazoline derivative having vinylamide bonding of the type represented by general formula (VII) can be produced in the same manner as below from I-1 in which either R² or R³ in general formula (I) is represented by general formula (II) above and the other is an iodine atom (route C).

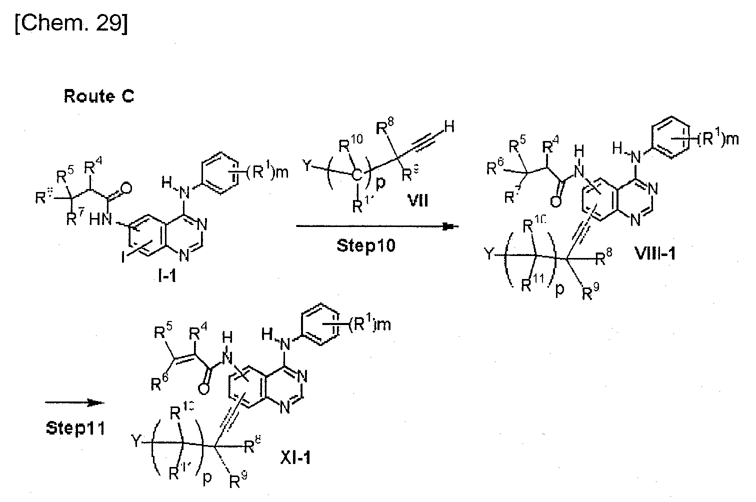

Step 10 is a step in which compound VIII-1 is produced by allowing the iodine atom of compound I-1 to react with compound VII using a Pd catalyst in the presence of a copper salt. The reaction is carried out in the presence of 0.001 to 1 equivalent, and preferably 0.001 to 0.2 equivalent, of a monovalent copper salt such as copper chloride, copper bromide, or copper iodide with respect to compound I-1, and in the presence of palladium conjugate prepared from 0 to 1 equivalent, and preferably 0 to 0.4 equivalent, of unidentate phosphine ligand such as triphenylphosphine, tributylphosphine, or tri-tert-butylphosphine, or bidentate phosphine ligand such as 1,1'-bis(diphenylphosphino)ferrocene, 1,3-bis(biphenyphosphino)propane, 1,2-bis(diphenylphosphino)ethane, 1,4-bis(diphenylphosphino)butane, (*R*)-(+)-2,2'-bis(diphenylphosphino)-1,1'-binaphthyl, or (S)-(-)-2,2'-bis(diphenylphosphino)-1,1'-binaphthyl on 0.001 to 1 equivalent, and preferably 0.001 to 0.2 equivalent, of 0-valent or divalent palladium catalyst such as palladium chloride, palladium acetate, palladium dichlorobis(benzonitrile)), dichlorobis(1,5-cyclooctadiene)palladium, di-µ-chlorobis(triphenylphosphine)dipalladium, tetrakis(triphenylphosphine)palladium, or tris(dibenzylidene acetone)dipalladium. Examples of bases suitable for the reaction include 0 to 100 equivalents, and preferably 1 to 10 equivalents, of a nitrogen-containing base such as triethylamine, diethylamine, or pyridine or inorganic bases such as sodium carbonate, potassium carbonate, or cesium fluoride, and regarding the solvent, compound VIII-1 may be derived by treating at -50 to 300°C, preferably 10 to 40°C, and more preferably 15 to 30°C, in a hydrocarbon-based solvent such as toluene, an ether-based solvent such as THF, methyltetrahydrofuran, or ethylene glycol, an ester-based solvent such as ethyl acetate or isopropyl acetate, an aprotic polar solvent such as DMF, 1,3-dimethyl-2-imidazolidinone, 1-methyl-2-pyrrolidone, DMSO, or acetonitrile, or an alcohol-based solvent such as ethanol, methanol, IPA, propanol, or butanol.

Conversion of compound VIII-1 to XI-1 is carried out under mild conditions of the type whereby polymerization of the vinylamide moieties of compound XI-1 can be suppressed. Auxiliary agents that are used are, for example, organic bases typified by triethylamine, diisopropylethylamine, pyridine, DABCO or the like, inorganic bases typified by potassium carbonate, potassium bicarbonate, potassium phosphate, potassium t-butoxide or the like, and inorganic or organic fluorides such as tetra-n-butylammonium fluoride, benzyltriethylammonium fluoride, tetraethylammonium fluoride, potassium fluoride, and cesium fluoride.

Compound XI-1 can be derived by means of treatment at -50 to 300°C, and preferably 10 to 60°C, in a hydrocarbon-based solvent such as toluene, an ether-based solvent such as THF, methyltetrahydrofuran, or ethylene glycol, an ester-based solvent such as ethyl acetate or isopropyl acetate, an aprotic polar solvent such as DMF, 1,3-dimethyl-2-imidazolidinone, 1-methyl-2-pyrrolidone, DMSO, or acetonitrile, or an alcohol-based solvent such as ethanol, methanol, IPA, propanol, or butanol.

### WORKING EXAMPLES

Working examples are presented below in order to facilitate understanding of the present invention, but without going beyond the gist thereof, the present invention is not limited to the following working examples.

Furthermore, production examples for the raw materials used in the present invention will be described before the working examples. When not otherwise specified, the respective procedures were as follows.

The reaction processes were carried out specifically at 18 to 25°C in an inert gas atmosphere (such as nitrogen).

Concentration of solutions was carried out using a rotary evaporator under reduced pressure.

Solvent drying was carried out on, for example, anhydrous sodium sulfate, and the desiccant was removed by filtration.

Column chromatography was carried out using a suitable developing solution, such as chloroform-methanol.

The structure of the target product was confirmed by proton nuclear magnetic resonance (¹H-NMR; 400 MHz). ¹H-NMR, when not otherwise specified, involved measurement in deuterated DMSO (DMSO-d₆) or deuterated chloroform (CDCl₃) with the chemical shift values expressed as δ values (ppm) using tetramethylsilane (TMS) as a standard and multiple peaks were indicated as described below.

s denotes singlet, d denotes doublet, t denotes triplet, m denotes multiplet, br denotes broad peak, and the magnitude of signal splitting is represented in units of Hz using the coupling constant J as a symbol.

### (PRODUCTION EXAMPLE 1) 7-iodo-6-nitro-4 (3H)-quinazoline: Compound (1)

7-chloro-6-nitro-4 (3H)-quinazolinone (50.0 g, 222 mmol) and sodium iodide (332 g, 2216 mmol) were added to N-methylpyrrolidone (500 mL), and were stirred for 5 h at 170°C.

The reaction solution was poured into water (1500 mL) and was stirred for 1 h.

The precipitated crystals were collected by filtration, and after washing with water, were dried under reduced pressure to obtain 7-iodo-6-nitro-4 (3H)-quinazolinone (1) (44.7 g, 64%).
¹H-NMR (DMSO-d₆) ppm: 8.3 (s, 1 H), 8.3 (s, 1 H), 8.5 (s, 1 H), 12.7 (brs, 1 H)

### (WORKING EXAMPLE 1) 7-iodo-3-(4-methoxybenzyl)-6-nitro-4-quinazolinone: Compound (2)

DMF (30 mL) and triethylamine (1.4 mL, 10.5 mmol) were added to compound (1) of Production Example 1 (3.17 g, 10 mmol), and after stirring, a solution of 4-methoxybenzyl chloride (1.64 g, 10.5 mmol) in DMF (5 mL) was added; after stirring for 22 h, the temperature was increased to 50°C, and this was stirred for an additional 6 h. Water (70 mL) was added to the reaction solution, the precipitated crystals were collected by filtration, and after washing with ethanol/water (2/1), dried under reduced pressure to obtain the target compound (2) (3.91 g, 89%).
¹H-NMR (DMSO-d₆) ppm: 3.7 (s, 3H), 5.1 (s, 2H), 6.9 (d, 2H, J=9.0 Hz), 7.3 (d, 2H, J=9.0 Hz), 8.4 (s, 1H), 8.6 (s, 1H), 8.7 (s, 1 H)

### (WORKING EXAMPLE 2) 6-amino-7-iodo-3-(4-methoxybenzyl)-4-quinazoline: Compound (3)

Ethanol (40 mL), activated carbon (grade: *Shirasagi,* purification, 0.16 g), and anhydrous iron chloride (7.5 mg, 0.046 mmol) were added to compound (2) of Working Example 1 (2.02 g, 4.62 mmol), and after heating to 65°C, hydrazine hydrate (584 mg, 9.33 mol) was added and this was stirred for 3 h. After allowing the reaction solution to cool, the precipitated crystals were collected by filtration and dried under reduced pressure to obtain target compound (3) (1.89 g, weight including activated carbon).
¹H-NMR (DMSO-d₆) ppm: 3.7 (s, 3H), 5.1 (s, 2H), 5.7 (s, 2H), 6.9 (d, 2H, J=9.0 Hz), 7.3 (d, 2H, J=9.0 Hz), 7.4 (s, 1 H), 8.0 (s, 1 H), 8.2 (s, 1 H)

### (WORKING EXAMPLE 3) N-{7-iodo-3-(4-methoxybenzyl)-4-oxo-3,4-dihydro-6-quinazolinyl}-3-(phenylsulfonyl)propanamide: Compound (4)

DMF (20 mL), 3-phenylsulfonylpropionic acid (1.04 g, 4.83 mmol), and n-propylphosphonic anhydride, cyclic trimer (50% ethyl acetate solution; 5.39 g, 8.463 mmol) were added, while chilling on ice, to compound (3) of Working Example 2 (1.80 g, including the weight of activated carbon), triethylamine (1.13 mL, 8.06 mmol) was added dropwise, and this was stirred for 2 h; 3-phenylsulfonylpropionic acid (1.04 g, 4.83 mmol), n-propylphosphonic anhydride, cyclic trimer (50% ethyl acetate solution; 5.39 g, 8.463 mmol), and triethylamine (1.13 mL, 8.06 mmol) were again added, and after stirring for 2 h, the reaction solution was heated to 35°C and stirred for 2 h. The reaction solution was filtered, a 10% potassium bicarbonate solution (30 mL) was added to the filtrate and stirred, and the precipitated crystals were filtered. The crystals were washed in suspension with acetonitrile/water (1/1; 30 mL), and were then washed in suspension with acetonitrile (30 mL) while heating under reflux; water (10 mL) was added, and after stirring for 15 min at 20°C, the precipitated crystals were filtered and dried under reduced pressure to obtain target compound (4) (1.80 g, yield 68% from compound (2) of Working Example 1).
¹H-NMR (DMSO-d₆) δ ppm: 2.9 (t, 2H, J=7.5 Hz), 3.6 (t, 2H, J=7.5 Hz), 3.7 (s, 3H), 5.1 (s, 2H), 6.9 (d, 2H, J=8.0 Hz), 7.3 (d, 2H, J=8.0 Hz), 7.7 (m, 2H), 7.8 (m, 1 H), 8.0 (d, 2H, J=8.0 Hz), 8.0 (s, 1 H), 8.2 (s, 1 H), 8.5 (s, 1 H), 9.8 (s, 1 H)

### (WORKING EXAMPLE 4) N-(7-iodo-4-oxo-3,4-dihydro-6-quinazolinyl)-3-(phenylsulfonyl)propanamide: Compound (5)

Compound (4) of Working Example 3 (1.20 g, 1.99 mmol) was dissolved in trifluoroacetic acid (7 mL), and was stirred for 30 min while heating at reflux. The reaction solution was concentrated, acetonitrile (5 mL) and water (5 mL) were added, and this was stirred for 1 h at room temperature.

The precipitated crystals were collected by filtration, and were dried under reduced pressure to obtain 1.04 g of crystals.

Acetonitrile/water (1/1; 10 m L) was added to 1.01 g of the crystals, triethylamine (0.4 mL) was added, and after stirring for 30 min, the precipitated crystals were collected by filtration and dried under reduced pressure to obtain the target compound (5) (0.74 g, 79%).
¹H-NMR (DMSO-d₆) δ ppm: 2.8 (t, 2H, J=7.5 Hz), 3.6 (t, 2H, J=7.5 Hz), 7.7 (t, 2H, J=7.5 Hz), 7.8 (t, 1H, J=7.5 Hz), 8.0 (d, 2H, J=7.5 Hz), 8.0 (s, 1 H), 8.1 (s, 1 H), 8.2 (s, 1 H), 9.8 (s, 1 H), 12.3 (brs, 1H)

### (WORKING EXAMPLE 5) N-{4-{(3-Chloro-4-fluorophenyl)amino}-7-iodo-6-quinazolinyl}-3-(phenylsulfonyl)propanamide: Compound (6) Hydrochloride

Toluene (2 mL), phosphorus oxychloride (55 mg, 0.354 mmol), and N,N'-diisopropylethylamine (36 mg, 0.281 mmol) were added to compound (5) of Working Example 4 (68 mg, 0.141 mmol), and after stirring at room temperature, 1,2-dichloroethane (2 mL) was added and stirred for 4 h at 70°C. After cooling the reaction solution, the insoluble matter was filtered out, the filtrate was concentrated, and methylene chloride (1 mL), toluene (1 mL), and a solution of 3-chloro-4-fluoroaniline (100 mg, 0.687 mmol) in IPA (1 mL) were added. After stirring for 3 h at room temperature, n-hexane (2 mL) was added and stirred for 1 h, and the precipitated crystals were filtered and dried under reduced pressure to obtain hydrochloride of target compound (6) (28 mg, 31%).
¹H-NMR (DMSO-d₆) δ ppm: 2.8 (m, 2H), 3.7 (t, 2H, J=8.0 Hz), 7.5 (t, 1H, J=9.0 Hz), 7.7 (m, 2H), 7.8 (t, 1H, J=7.5 Hz), 8.0 (d, 2H, J=7.5 Hz), 8.1 (dd, 1H, J=3.0, 7.0 Hz), 8.4 (s, 1 H), 8.6 (s, 1 H), 8.8 (s, 1 H), 10.2 (s, 1 H), 11.1 (brs, 1 H)

### (WORKING EXAMPLE 6) 4-(3-Chloro-4-fluoroanilino)-7-iodo-6-nitroquinazoline: Compound (15-1)

Compound (1) of Production Example 1 (30.0 g, 94.6 mmol) was added to toluene (450 mL), and phosphorus oxychloride (13.2 mL, 142 mmol) and N,N-diisopropylethylamine (33.0 mL, 189 mmol) were added to this suspension, whereupon this was stirred for 12 h at 75°C. The reaction solution was cooled to 2°C, a solution of 3-chloro-4-fluoroaniline (16.5 g, 114 mmol) in IPA (175 mL) was added dropwise, and this was stirred for 1 h. N-heptane (150 mL) was added at 2°C, and was stirred for an additional 3 hours. The precipitated crystal were filtered and washed with toluene (90 mL x 2). The resulting crystals were then washed in a suspension produced using acetonitrile (600 mL) heated to 80°C, and the crystals were filtered and washed with acetonitrile (90 mL x 2). The resulting crystals were added to acetonitrile (300 mL)-water (300 ml), and triethylamine (19.8 mL, 142 mmol) was added dropwise at room temperature. Stirring was then carried out for 1 h at room temperature. The crystals were filtered and washed with water (90 mL x 2). Upon drying under reduced pressure, the target compound (15-1) was obtained as brown crystals (32.2 g, 76%).
¹H-NMR (DMSO-d₆) δ ppm: 7.46 (t, 1H, J=8.8 Hz), 7.79 (m, 1 H), 8.14 (dd, 1 H, J=2.4, 6.8 Hz), 8.44 (s, 1 H), 8.72 (s, 1 H), 9.22 (s, 1H), 10.30 (br s, 1 H)

### (WORKING EXAMPLE 7) N-4-(3-Chloro-4-fluorophenyl)-7-iodo-4,6-quinazoline diamine: Compound (15-2)

Compound (15-1) of Working Example 6 (80.0 g, 180 mmol), anhydrous ferric chloride (III)(292 mg, 1.80 mmol) and activated carbon (grade: Shirasagi, purification, 22.5 g) were mixed in methanol (1600 mL), and hydrazine hydrate (80%; 21.9 mL, 360 mmol) was added dropwise at 64°C. This was stirred for 2 h at 64°C, THF (960 mL) was added at room temperature, and the activated carbon was filtered out. About 1800 mL of the solvent was evaporated off under reduced pressure, methanol (1200 mL) was added, and about 1200 mL of the solvent was evaporated off under reduced pressure. This was stirred for 2 h while chilling on ice, and the precipitated crystals were filtered and washed with methanol (240 mL). Upon drying under reduced pressure, target compound (15-2) was obtained as yellow-white crystals (69.4 g, 93%).
¹H-NMR (DMSO-d₆) δ ppm: 5.60 (br s, 2H), 7.42 (t, 1 H, J=8.8 Hz), 7.52 (s, 1 H), 7.80 (ddd, 1 H, J=2.4, 6.8, 8.8 Hz), 8.18 (dd, 1 H, J=2.4, 6.8 Hz), 8.19 (s, 1H), 8.38 (s, 1 H), 9.67 (s, 1 H)

### (WORKING EXAMPLE 8) N-{4-{(3-chloro-4-fluorophenyl)amino}-7-iodo-6-quinazolinyl}-3-(phenylsulfonyl)propanamide: Compound (6)

3-phenylsulfonylpropionic acid (21.7 g, 101 mmol) and n-propanephosphonic anhydride, cyclic trimer (50% ethyl acetate solution; 100 mL, 169 mmol) were added to a solution of compound (15-2) of Working Example 7 (35.0 g, 84.4 mmol) in THF (350 mL), and triethylamine (22.4 mL, 160 mmol) was added dropwise at 3°C. The reaction solution was warmed to room temperature, and was stirred for 12 h at room temperature. 2-propanol (175 mL) was added, water (525 mL) and triethylamine (47.1 mL, 338 mmol) were then added, and this was stirred for 2 h at 3°C. The precipitated crystals were filtered and washed with THF/water = 1/1 (105 mL x 2). Upon drying under reduced pressure, the target compound (6) was obtained as milky-white crystals (50.6 g, 98%).
¹H-NMR (DMSO-d₆) δ ppm: 2.81 (t, 2H, J=7.2 Hz), 3.67 (t, 2H, J=7.2 Hz), 7.44 (t, 1H, J=8.8 Hz), 7.77 (m, 4H), 7.99 (m, 2H), 8.13 (dd, 1 H, J=2.4, 6.4 Hz), 8.36 (s, 1 H), 8.43 (s, 1 H), 8.60 (s, 1 H), 10.02 (s, 1 H), 10.11 (s, 1 H)

### (WORKING EXAMPLE 9) N-{4-(3-chloro-4-fluorophenyl)amino]-7-[3-methyl-3-(4-methyl-1-piperazinyl)-1-butynyl]-6-quinazolinyl}-3-(phenylsulfonyl)propanamide: Compound 8

A solution of palladium (II) acetate (110 mg, 0.49 mmol) and triphenylphosphine (258 mg, 0.98 mmol) in DMSO (100 mL) was stirred for 30 min at room temperature, whereupon compound (6) of Working Example 5 (10.0 g, 16.4 mmol), 1-(1,1-dimethyl-2-propynyl)-4-methylpiperazine (7) (3.27 g, 19.6 mmol) and copper (I) iodide (93.0 mg, 0.49 mmol) were added, and triethylamine (5.70 mL, 40.9 mmol) was added dropwise. After stirring for 3 h, the reaction solution was added dropwise to water (100 mL), and was stirred for one additional hour. The precipitated crystals were filtered and washed with water, before washing the crystals as a suspension in IPA/water (1/1; 200 mL).

The precipitated crystals were filtered and dried under reduced pressure to obtain target compound (8) (yield of 96% from compound (6) of Working Example 5, yield of 80% from compound (7)).
¹H-NMR (DMSO-d₆) δ ppm: 1.4 (s, 6H), 2.2 (s, 3H), 2.3 (brs, 4H), 2.6 (brs, 4H), 2.8 (t, 2H, J=7.5 Hz), 3.7 (t, 2H, J=7.5 Hz), 7.5 (t, 1H, J=9.0 Hz), 7.7 (m, 5H), 8.0 (m, 2H), 8.2 (dd, 1H, J=3.0, 7.0 Hz), 8.50 (s, 1 H), 8.62 (s, 1 H), 9.87 (s, 1 H), 9.96 (s, 1 H)

### (WORKING EXAMPLE 10) N-{[(3-chloro-4-fluorophenyl)amino]-7-[3-methyl-3-(4-methyl-1-piperazinyl)-1-butynyl]-6-quinazolinyl}acrylamide: Compound (9)

Tetra-n-butylammonium fluoride (1 mol/L, THF solution; 46.2 mL, 46.2 mmol) was added dropwise to a solution of compound (8) of Working Example 9 (5.00 g, 7.70 mmol) in THF (100 mL). After stirring for 12 h, a 10% potassium bicarbonate aqueous solution (50 mL) was added to the reaction solution, and decanted. The organic layer was washed with 10% potassium bicarbonate aqueous solution (50 mL), and was then washed with 10% sodium chloride aqueous solution (50 ml). IPA (100 mL) was added to the organic layer, and 100 mL of the solvent was then evaporated off under reduced pressure. Next, water (100 ml) was added dropwise, and after stirring for 1 h while chilling on ice, the precipitated crystals were filtered, washed twice with IPA/water (1/1; 20 mL), and dried under reduced pressure to obtain target compound (9) (3.24 g, 83%).
¹H-NMR (DMSO-d₆) δ ppm: 1.4 (s, 6H), 2.2 (s, 3H), 2.4 (brs, 4H), 2.6 (brs, 4H), 5.8 (d, 1H, J=11.0 Hz), 6.3 (d, 1H, J=17.0 Hz), 6.6 (dd, 1H, J=11.0, 17.0 Hz), 7.5 (t, 1H, J=9.0 Hz), 7.8 (m, 2H), 8.2 (dd, 1H, J=2.0, 7.0 Hz), 8.6 (s, 1 H), 8.7 (s, 1 H), 8.9 (s, 1 H), 10.0 (s, 1 H)

### (WORKING EXAMPLE 11) N-{[(3-chloro-4-fluorophenyl)amino]-7-[3-methyl-3-(4-methyl-1-piperazinyl)1-butynyl]-6-quinazolinyl}acrylamide: Compound (9)

Potassium tert-butoxide (28.0 mg, 0.25 mmol) was added at room temperature to a solution of N-{4-[(3-chloro-4-fluorophenyl)amino]-7-[3-methyl-3-(4-methyt-1-piperazinyl)-1-butynyl]-6-quinazolinyl}-3-(phenyloxy)propanamide: compound (8-2) (50.0 mg, 0.08 mmol) synthesized in the same manner as compound (8) of Working Example 9 in DMF (1 mL), and was stirred for 1 h at room temperature.

Ethyl acetate (10 mL) and 10% sodium chloride aqueous solution (5 mL) were added to the reaction solution, and decanting was performed. The aqueous layer was subjected to extraction using ethyl acetate (5 mL). The organic layers were mixed, and were then washed with 10% sodium chloride aqueous solution (5 mL x 2). After drying on anhydrous sodium sulfate, the solvent was evaporated off under reduced pressure, and the residue was purified by silica gel column chromatography (methylene chloride → methylene chloride/methanol = 4/1) to obtain N-{[(3-chloro-4-fluorophenyl)amino]-7-[3-methyl-3-(4-methyl-1-piperazinyl)1-butynyl]-6-quinazolinyl}acrylamide compound (9) (20.0 mg, 47%) as yellow crystals.
(Reference Example 1) Synthesis of N-{4-[(3-chloro-4-fluorophenyl)amino]-7-[3-methyl-3-(4-methyl-1-piperazinyl)-1-butynyl]-6-quinazolinyl}-3-(phenylsulfonyl)propanamide, compound (8), by means of a reaction between 1-(1,1-dimethyl-2-propynyl)-4-methylpiperazine, compound (7), with N-{4-[(3-chloro-4-fluorophenyl)amino]-7-iodo-6-quinazolinyl}-3-(phenylsulfonyl)propanamide compound (6), N-{4-[(3-chloro-4-fluorophenyl)amino]-7-bromo-6-quinazolinyl}-3-(phenylsulfonyl)propanamide, compound (6-1), or N-{4-[(3-chloro-4-fluorophenyl)amino]-7-chloro-6-quinazolinyl}-3-(phenylsulfonyl)propanamide, compound (6-2)

Each of the reactions was carried out under the same conditions as in Working Example 9, and analysis was carried out under the HPLC conditions indicated below after sampling the respective reaction solutions at a determinate time.

**(TABLE 1)**

| HPLC conditions |
|---|
| Column: L-column ODS diameter 4.6 mm x 250 mm |
| Mobile layer: 0.1 % trifluoroacetic acid acetonitrile solution/0.1 % trifluoroacetic acid aqueous solution = 40/60 (volume ratio) |
| Flow rate: 1 mL/min |
| Detection wavelength: 254 nm |
| Retention time: Compound (6) 11.2 min, Compound (6-1) 11.3 min, Compound (6-2) 11.2 min, 8 6.2 min |

| HPLC analysis results |
|---|
| HPLC percent surface area resulting from the reaction of compound (6) and compound (7): |
| After reacting for 1 h: Compound (6) 5.3%, Compound (8) 91.2% |
| After reacting for 3 h: Compound (6) not detected by HPLC, Compound (8) 94.5% |
| Compound (8) obtained by treatment using a procedure analogous to Working Example 6 after reacting for 3 h: Isolated yield 96%. |
| HPLC percent surface area resulting from the reaction of compound (6-1) and compound (7): |
| After reacting for 1 h: Compound (6-1) 58.9%, Compound (8) 38.7% |
| After reacting for 23 h: Compound (6-1) 40.3%, Compound (8) 55.7% |
| HPLC percent surface area resulting from the reaction of compound (6-2) and compound (7): |
| After reacting for 1 h: Compound (6-2) 98.2%, Compound (8) not detected by HPLC |
| After reacting for 10 h: Compound (6-2) 96.3%, Compound (8) 0.2% |

As described above, iodoquinazoline compound (6) exhibited high reactivity in the carbon-carbon bond production reaction using Pd catalyst in comparison to the corresponding bromo compound (6-1) or chloro compound (6-2).

### INDUSTRIAL APPLICABILITY

By virtue of the present invention, a novel quinazoline derivative can be provided for use as an intermediate in the production of pharmaceutical products such as cancer drugs. Furthermore, the quinazoline derivative can be used in order to provide an efficient production method for pharmaceutical products.

Note that this application was filed claiming priority from JP-2003-398442.

## Claims

1. A quinazoline derivative represented by general formula (I) below, or a salt thereof, or a hydrate or solvate thereof: 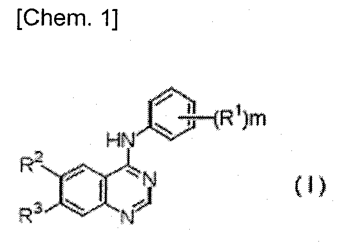 [in the formula, m denotes an integer from 0 to 3, R¹ denotes a hydrogen atom, halogen atom, hydroxy group, cyano group, nitro group, trifluoromethyl group, C₁ to C₅ alkyl group, C₁ to C₅ alkoxy group, -S(O)_{f}R¹² (in the formula, f denotes an integer from 0 to 2, R¹² denotes a C₁ to C₅ alkyl group), -NR¹³R¹⁴ (in the formula, R¹³ and R¹⁴ each individually denotes a hydrogen atom, C₁ to C₅ alkyl group, C₁ to C₅ alkanoyl group, or C₁ to C₅ alkylsulfonyl group), C₂ to C₅ alkenyl group, or C₂ to C₅ alkynyl group, and either one of R² and R³ denotes general formula (II) below 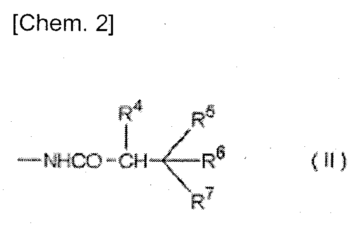 (in the formula, R⁴, R⁵ and R⁶ each individually denotes a hydrogen atom, C₁ to C₅ alkyl group that may have substituents, C₇ to C₁₂ aralkyl group that may have substituents, or C₆ to C₁₀ aryl group that may have substituents, R⁷ denotes -SO₂R¹⁵, -SOR¹⁵, or -OR¹⁵ (in the formula, R¹⁵ denotes a C₁ to C₅ alkyl group that may have substituents, C₇ to C₁₂ aralkyl group that may have substituents, or C₆ to C₁₀ aryl group that may have substituents) and the remaining one of R² and R³ denotes an iodine atom or general formula (III) below: 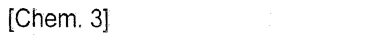 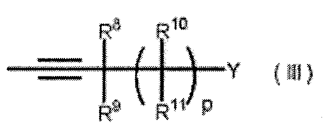 (in the formula, R⁸ and R⁹ each individually denotes a hydrogen atom, or a C₁ to C₅ alkyl group that may be substituted with a hydroxyl group or C₁ to C₅ alkoxy group, p denotes an integer from 0 to 3, R¹⁰ and R¹¹ each individually denotes a hydrogen atom or C₁ to C₅ alkyl group, Y denotes a hydrogen atom, hydroxyl group, C₁ to C₅ alkoxy group, C₁ to C₅ alkanoyloxy group, piperazin-1-yl that has a C₁ to C₅ alkyl group that may be substituted at the 4-position, or an amino that is di-substituted with C₁ to C₅ alkyls that may be substituted), and herein, when m denotes 2 or 3, R¹ may be the same or different.]

2. The quinazoline derivative, salt thereof, or hydrate or solvate thereof according to Claim 1, wherein m is 2, R¹ is a halogen atom, R² is -NHCO-CH₂-CH₂-R⁷ (in the formula, R⁷ denotes a methylsulfonyl group, benzenesulfonyl group, phenyloxy group, phenylthio group, or methylthio group), and R³ is an iodine atom or general formula (IV) below: 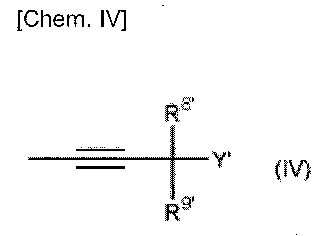 (in the formula, R^{8'} and R^{9'} each individually denotes a hydrogen atom, methyl group, ethyl group, propyl group, or isopropyl group, and Y' denotes a morpholino group or 4-methylpiperazin-1-yl).

3. The quinazoline derivative, salt thereof, or hydrate or solvate thereof according to either Claim 1 or 2, selected from a group consisting of the following compounds:
N-{4-[(3-chloro-4-fluorophenyl)amino]-7-iodo-6-quinazolinyl}-3-(phenylsulfonyl)propanamide, N-{4-[(3-chloro-4-fluorophenyl)amino]-7-iodo-6-quinazolinyl}-3-(phenyloxy)propanamide, N-{4-[(3-chloro-4-fluorophenyl)amino]-7-[3-methyl-3-(4-methyl-1-piperazinyl)-1-butynyl]-6-quinazolinyl}-3-(phenylsulfonyl)propanamide, N-{4-[(3-chloro-4-fluorophenyl)amino]-7-[3-methyl-3-(4-methyl-1-piperazinyl)-1-butynyl]-6-quinazolinyl}-3-(phenyloxy)propanamide.

4. The quinazoline derivative, salt thereof, or hydrate or solvate thereof according to Claim 3, wherein the compound is N-{4-[(3-chloro-4-fluorophenyl)amino]-7-iodo-6-quinazolinyl}-3-(phenylsulfonyl)propanamide.

5. A method for preparing the quinazoline derivative represented by general formula (I) of Claim 1 [where either of R² and R³ denotes general formula (II) of Claim 1, and the other of R² and R³ denotes general formula (III) of Claim 1], salt thereof, or hydrate or solvate thereof, by allowing the quinazoline derivative represented by general formula (V) below: 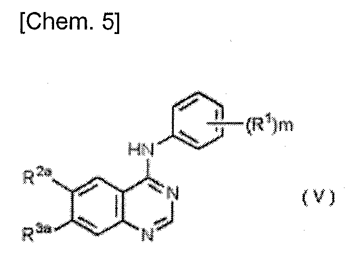 [in the formula, m and R¹ are the same as in Claim 1, either one of R^{2a} and R^{3a} is defined the same as in general formula (II) of Claim 1, and the other of R^{2a} and R^{3a} denotes an iodine atom],
or salt thereof, or hydrate or solvate thereof to react with a compound represented by general formula (VI) below: 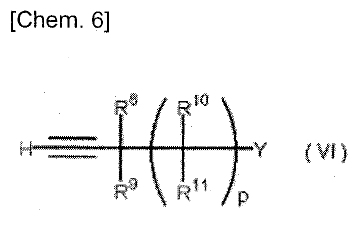 (in the formula, R⁸, R⁹, R¹⁰, R¹¹, Y and p are defined the same as in Claim 1),
or salt thereof, or hydrate or solvate thereof.

6. The preparation method according to Claim 5, wherein m is 2, R¹ is a halogen atom, R^{2a} is - NHCO-CH₂-CH₂-R⁷ (in the formula, R⁷ denotes a methylsulfonyl group, benzenesulfonyl group, phenyloxy group, phenylthio group, or methylthio group), and R³ is an iodine atom.

7. The preparation method according to Claim 5, wherein the quinazoline derivative represented by general formula (V) is N-{4-[(3-chloro-4-fluorophenyl)amino]-7-iodo-6-quinazolinyl}-3-(phenylsulfonyl)propanamide or N-{4-[(3-chloro-4-fluorophenyl)amino]-7-iodo-6-quinazolinyl}-3-(phenyloxy)propanamide.

8. The preparation method according to Claim 7, wherein the quinazoline derivative is N-{4-[(3-chloro-4-fluorophenyl)amino]-7-iodo-6-quinazolinyl}-3-(phenylsulfonyl)propanamide.

9. A method for preparing the compound represented by general formula (III) of Claim 1, a pharmaceutically acceptable salt thereof, or a hydrate or solvate thereof, using any of the compounds recited in Claim 1-4, represented by general formula (VII) below: 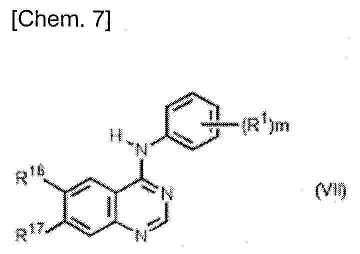 [in the formula, m and R¹ are defined the same as in Claim 1, either one of R¹⁶ and R¹⁷ denotes -NHCO-CR⁴=CR⁵R⁶ (in the formula, R⁴, R⁵, and R⁶ are defined the same as in Claim 1), and the other one of R¹⁶ and R¹⁷ is.

10. The preparation method according to Claim 9, wherein m is 2, R¹ is a halogen, R² is - NHCO-CH₂CH₂-R⁷, R¹⁶ is -NHCO-CH=CH₂, and R³ and R¹⁷ are general formula (IV) of Claim 2.

11. The preparation method according to Claim 10, wherein R^{8'} and R^{9'} each individually is a methyl group, and Y' is 4-methylpiperazin-1-yl.

12. The preparation method for the compound represented by general formula (VII) of Claim 9, salt thereof, or hydrate or solvate thereof comprising the preparation method according to any of Claims 5 to 11.

13. The preparation method according to Claim 12, wherein m is 2, R¹ is a halogen, R² is - NHCO-CH₂CH₂-R⁷, R¹⁶ is -NHCO-CH=CH₂, and R³ and R¹⁷ are general formula (IV) of Claim 2.

14. The preparation method according to Claim 12, wherein R^{8'} and R^{9'} each individually is a methyl group, and Y' is 4-methylpiperazin-1-yl.

15. The compound represented by general formula (VIII) below: 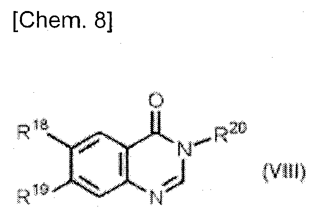 [in the formula, either of R¹⁸ and R¹⁹ denotes a nitro group, amino group, hydroxyamino group, or -NHCO-CH₂CH₂-R^{7'} (in the formula, R^{7'} denotes a methylsulfonyl group, benzenesulfonyl group, phenyloxy group, phenylthio group, or methylthio group), and the remaining one of R¹⁸ and R¹⁹ denotes an iodine atom, and R²⁰ denotes a hydrogen atom, 3,4-dimethoxybenzyl group, 4-methoxybenzyl group, benzyloxymethyl group, or trifluoroacetyl group],
a salt thereof, or a hydrate or solvate thereof.

16. A compound, salt thereof, or hydrate or solvate thereof according to Claim 15, selected from a group consisting of the following compounds:
7-iodo-3-(4-methoxybenzyl)-6-nitro-4-quinazolinone, 6-amino-7-iodo-3-(4-methoxybenzyl)-4-quinazolinone, N-[7-iodo-3-(4-methoxybenzyl)-4-oxo-3,4-dihydro-6-quinazolinyl]-3-(phenylsulfonyl)propanamide, N-[7-iodo-3-(4-methoxybenzyl)-4-oxo-3,4-dihydro-6-quinazolinyl]-3-(phenyloxy)propanamide, N-(7-iodo-4-oxo-3,4-dihydro-6-quinazolinyl)-3-(phenylsulfonyl)propanamide, and N-(7-iodo-4-oxo-3,4-dihydro-6-quinazolinyl)-3-(phenyloxy)propanamide.

17. The compound, salt thereof, or hydrate or solvate thereof according to Claim 16, wherein the compound is 7-iodo-3-(4-methoxybenzyl)-6-nitro-4-quinazolinone, 6-amino-7-iodo-3-(4-methoxybenzyl)-4-quinazolinone, N-[7-iodo-3-(4-methoxybenzyl)-4-oxo-3,4-dihydro-6-quinazolinyl]-3-(phenylsulfonyl)propanamide, or N-(7-iodo-4-oxo-3,4-dihydro-6-quinazolinyl)-3-(phenylsulfonyl)propanamide.

18. The preparation method for the compound of general formula (I) in Claim 1 which uses any of the compounds according to any of Claims 15 to 17.

19. The preparation method according to Claim 18, wherein m is 2, R¹ is a halogen atom, R² is - NHCO-CH₂-CH₂-R⁷ (in the formula, R⁷ denotes a methylsulfonyl group, benzenesulfonyl group, phenyloxy group, phenylthio group, or methylthio group), and R³ is an iodine atom or general formula (IV) below: 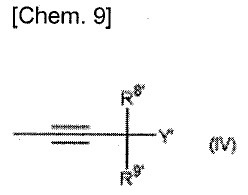 (in the formula, R^{8'} and R^{9'} each individually denotes a hydrogen atom, methyl group, ethyl group, propyl group, or isopropyl group, and Y' denotes a morpholino group or 4-methylpiperazin-1-yl).

20. The preparation method according to Claim 18, wherein the compound is N-{4-[(3-chloro-4-fluorophenyl)amino]-7-iodo-6-quinazotinyl}-3-(phenylsulfonyl)propanamide, N-{4-[(3-chloro-4-fluorophenyl)amino]-7-iodo-6-quinazolinyl}-3-(phenyloxy)propanamide, N-{4-[(3-chloro-4-fluorophenyl)amino]-7-[3-methyl-3-(4-methyl-1-piperazinyl)-1-butynyl]-6-quinazolinyl}-3-(phenylsulfonyl)propanamide, or N-{4-[(3-chloro-4-fluorophenyl)amino]-7-[3-methyl-3-(4-methyl-1-piperazinyl)-1-butynyl]-6-quinazolinyl}-3-(phenyloxy)propanamide.

21. The preparation method according to Claim 18, wherein the compound is N-{4-[(3-chloro-4-fluorophenyl)amino]-7-iodo-6-quinazolinyl}-3-(phenylsulfonyl)propanamide.

22. The method for preparing a compound represented by general formula (V) according to Claim 5, comprising a step in which a compound represented by general formula (IX) below: 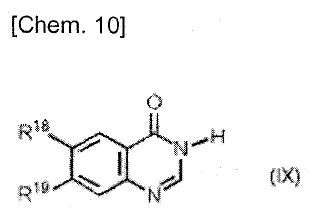 [in the formula, either one of R¹⁸ and R¹⁹ denotes general formula (II) of Claim 1, and the remaining one of R¹⁸ and R¹⁹ denotes an iodine atom]
is chlorinated to produce a compound represented by general formula (X) below: 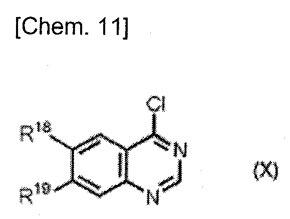 (in the formula, R¹⁸ and R¹⁹ are defined the same as above), and a step in which a compound represented by general formula (XI) below: 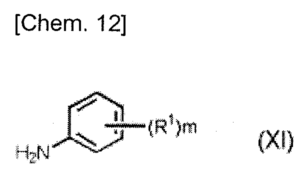 (in the formula, m and R¹ are the defined same as in Claim 1)
is added.

23. A quinazoline derivative represented by general formula (XII) below: 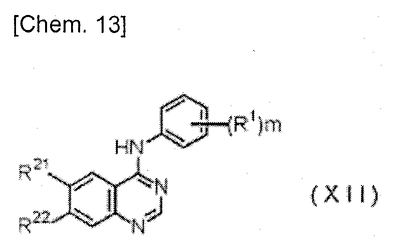 (in the formula, m and R¹ are defined the same as in Claim 1, either one of R²¹ and R²² denotes an amino group or nitro group, and the remaining one of R²¹ and R²² denotes an iodine atom), a salt thereof, or a hydrate or solvate thereof.

24. A method for preparing a compound represented by general formula (I) of Claim 1, a pharmaceutically acceptable salt thereof, a hydrate or solvate thereof, wherein the nitro group of a compound wherein either one of R²¹ and R²² in general formula (XII) of Claim 23 is a nitro group and the other one of R²¹ and R²² is an iodine atom is changed to an amino group, whereupon a reaction is allowed to occur with a compound of general formula (XIII) below: 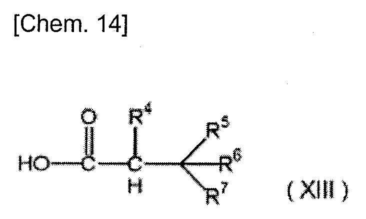 (in the formula, R⁴, R⁵, R⁶ and R⁷ are defined the same as in Claim 1).
